# EUROPEAN PATENT APPLICATION

(11) **EP 3 981 471 A1**
(43) Date of publication of application: **13.04.2022**
(21) Application number: 20818459.8
(22) Date of filing: 22.05.2020
(51) Int. Cl.: A61P 25/16, A61P 25/18, A61P 25/24, A61P 25/28, A61K 36/87, A23L 33/105, A23L 2/52, A61K 31/05, A61K 31/7028, C12G 1/00

(54) **GRAPE SKIN EXTRACT**

(30) Priority: 05.06.2019 JP 2019105648
(71) Applicant: University of Tsukuba, Ibaraki 305-8577 (JP); NATIONAL INSTITUTE OF ADVANCED INDUSTRIAL SCIENCE AND TECHNOLOGY, Chiyoda-ku Tokyo 100-8921 (JP); Zeon Corporation, Tokyo 100-8246 (JP)
(72) Inventor: ISODA Hiroko, Tsukuba-shi, Ibaraki 305-8577 (JP); SASAKI Kazunori, Tsukuba-shi, Ibaraki 305-8577 (JP); SATO Kazuhiko, Tsukuba-shi, Ibaraki 305-8561 (JP); ASAKAWA Masumi, Tsukuba-shi, Ibaraki 305-8561 (JP); TOMINAGA Kenichi, Tsukuba-shi, Ibaraki 305-8561 (JP); FUJISAWA Hiroshi, Tokyo 100-8246 (JP); KOSHIYAMA Masami, Tokyo 100-8246 (JP)
(74) Representative: Gerauer, Marc Philippé
(86) International application number: PCT/JP2020/020360
(87) International publication number: WO 2020/246272

(57) **Abstract**

Provided is a grape pericarp extract that exhibits neuronal function modulating effects. The grape pericarp extract comprises 15 mg/g or more of malvidin-3,5-diglucoside in dry mass.

## Description

### TECHNICAL FIELD

The present disclosure relates to grape pericarp extracts that comprise a specific amount of malvidin-3,5-diglucoside.

### BACKGROUND

Anthocyanins are known to exhibit various physiological activities, such as visual impairment improving effects, antioxidant effects, vascularization effects, anti-inflammatory effects, and anti-tumor effects. As such, attempts have been made to utilize biological resources containing anthocyanins.

For example, blueberry fruit extracts have been reported to be used as anti-cancer agents (PTL 1) and compositions for improving brain function (PTL 2). Attempts have also been made to suppress the aggregation of amyloid β protein by anthocyanins contained in bilberries (NPL 1).

### CITATION LIST

### Patent Literature

PTL 1: JP2003-277271A
PTL 2: JP2007-145825A

### Non-patent Literature

NPL 1: MY. Yoshida, et al. "Anthocyanin suppresses the toxicity of Aβ deposits through diversion of molecular forms in vitro and in vivo models of Alzheimer's disease" Nutritional Neuroscience, 2016, Vol. 19(1), p. 32-42

### SUMMARY

### (Technical Problem)

In Japan, the market for drugs for treating dementia continues to expand drastically as the number of patients with dementia has been increasing due to the aging population. The market size is expected to expand by 1.75 times in 2022 compared with 2013. Generally, medical treatments for dementia entail side effects and other adverse events, which impose a large burden on patients. On the other hand, substances derived from biological resources are safe with fewer side effects and hence are suitable for daily or continuous ingestion. Thus, there are high expectations for substances derived from biological resources that exhibit neuronal function modulating effects.

As to biological resources for anthocyanins, as mentioned above, blueberries and bilberries have been the center of research, and grapes and grape pericarps have not been sufficiently studied.

It would therefore be helpful to provide a grape pericarp extract that exhibits neuronal function modulating effects.

### (Solution to Problem)

The inventors focused on and studied grapes as a biological resource and established that a certain grape pericarp extract would exhibit superior neuronal function modulating effects. The inventors also established that a certain grape pericarp extract is rich in malvidin-3,5-diglucoside and is specific in the composition of anthocyanins. The present disclosure has been made based on the findings that a grape pericarp extract that exhibits superior neuronal function modulating effects can be obtained by controlling the malvidin-3,5-diglucoside content to fall within a specific range when extracting a grape pericarp.

### (Advantageous Effect)

According to the present disclosure, there is provided a grape pericarp extract that comprises 15 mg/g or more of malvidin-3,5-diglucoside in dry mass.

Malvidin-3,5-diglucoside is represented by the following formula (1):

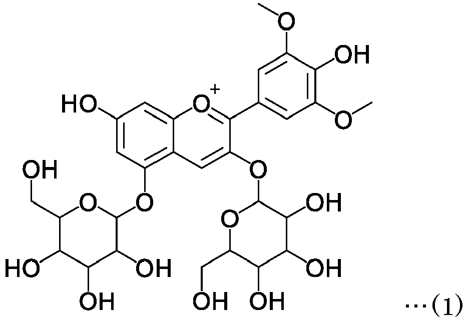

In the grape pericarp extract, malvidin-3,5-diglucoside may be in the form having a counter anion, e.g., chloride.

According to the present disclosure there is also provided the grape pericarp extract described above that comprises delphinidin-3-glucoside, and there is also provided the grape pericarp extract described above that comprises delphinidin-3-glucoside in an amount of 0.01 moles or more and 1.5 moles or less per mole of malvidin-3,5-diglucoside.

Delphinidin-3-glucoside is represented by the following formula (2):

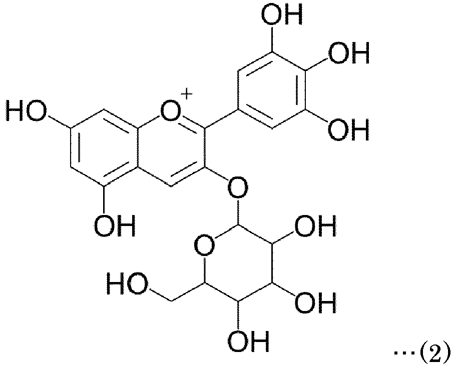

In the grape pericarp extract, delphinidin-3-glucoside may be in the form having a counter anion, e.g., chloride.

According to the present disclosure, there is also provided the grape pericarp extract described above that comprises malvidin-3-glucoside, and there is also provided the grape pericarp extract described above that comprises malvidin-3-glucoside in an amount of 0.01 moles or more and 1.5 moles or less per mole of malvidin-3,5-diglucoside.

Malvidin-3-glucoside is represented by the following formula (3):

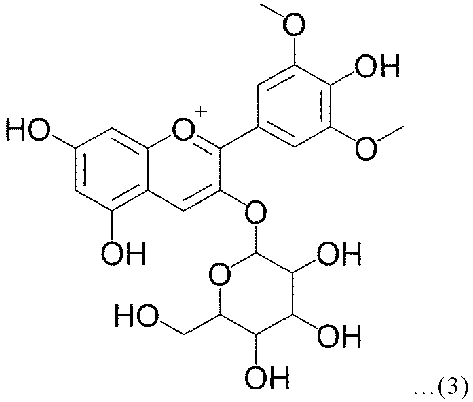

In the grape pericarp extract, malvidin-3-glucoside may be in the form having a counter anion, such as chloride.

According to the present disclosure, there is also provided the grape pericarp extract described above that comprises malvidin-3-coumaroylglucoside, and there is also provided grape pericarp extract described that comprises malvidin-3-coumaroylglucoside in an amount of 0.01 moles or more and 2.0 moles or less per mole of malvidin-3,5-diglucoside.

Malvidin-3-coumaroylglucoside is represented by the following formula (4):

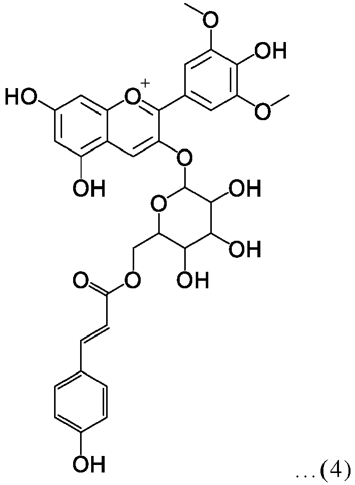

In the grape pericarp extract, malvidin-3-coumaroylglucoside may be in the form having a counter anion, e.g., chloride.

According to the present disclosure, there is also provided the grape pericarp extract described above that comprises malvidin-3-coumaroylglucoside-5-glucoside, and there is also provided the grape pericarp extract described above that comprises malvidin-3-coumaroylglucoside-5-glucoside in an amount of 0.01 moles or more and 2.0 moles or less per mole of malvidin-3,5-diglucoside.

Malvidin-3-coumaroylglucoside-5-glucoside is represented by the following formula (5):

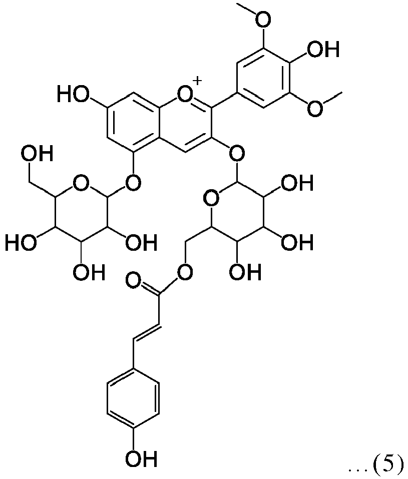

In the grape pericarp extract, malvidin-3-coumaroylglucoside-5-glucoside may be in the form having a counter anion, e.g., chloride.

According to the present disclosure, there is also provided the grape pericarp extract described above that comprises resveratrol, and there is also provided the grape pericarp extract described above that comprises resveratrol in an amount of 0.001 moles or more per mole of malvidin-3,5-diglucoside.

Resveratrol is represented by the following formula (6):

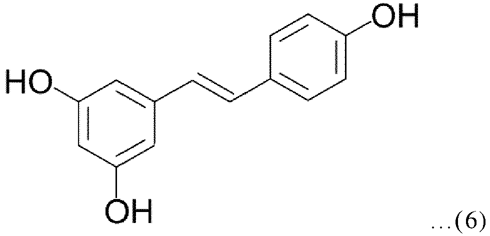

According to the present disclosure, there is also provided the grape pericarp extract of any of the foregoing, wherein the grape pericarp is a pericarp of one or more types of grapes selected from the group consisting of *Vitis Coignetiae, Vitis flexuosa,* a cross species between *Vitis Coignetiae* and *Vitis flexuosa,* a cross species between *Vitis Coignetiae* and *Vitis Vinifera,* and a cross species between *Vitis flexuosa* and *Vitis Vinifera.*

According to the present disclosure, there is also provided a food composition that comprises any of the grape pericarp extracts described above, and there is also provided the food composition described above, which is a beverage having an alcohol by volume of less than 3%

According to the present disclosure, there is also provided a pharmaceutical composition that comprises the grape pericarp extract of any of the foregoing.

According to the present disclosure, there is also provided a method of producing the grape pericarp extract described above, wherein an ingredient comprising malvidin-3,5-diglucoside is extracted from a grape pericarp using an organic solvent, and there is also provided the method of producing the grape pericarp extract described above, wherein a dried grape pericarp product is used as the grape pericarp and extraction is performed using either an acid-containing organic solvent or a mixed solvent of an acid-containing organic solvent and water.

### (Advantageous Effect)

The grape pericarp extract of the present disclosure exhibits superior neuronal function modulating effects. Further, according to the present disclosure, such a grape pericarp extract can be produced by a simple method.

### BRIEF DESCRIPTION OF THE DRAWINGS

In the accompanying drawings:
FIG. 1 illustrates a graph showing the results of the cell survival experiment in Example 1;
FIG. 2 is a graph showing the results of the animal experiment (experiment for confirming anti-inflammatory effects based on the expression of tumor necrosis factor (TNF)-α) in Example 2;
FIG. 3 illustrates a graph showing the results of the animal experiment (experiment for confirming anti-inflammatory effects based on the expression of interleukin 6 (IL-6)) in Example 2;
FIG. 4 illustrates a graph showing the results of the animal experiment (experiment for confirming inhibitory effects on neurotransmitter level reduction based on the noradrenaline level) in Example 3;
FIG. 5 illustrates a graph showing the results of the animal experiment (experiment for confirming inhibitory effects on neurotransmitter level reduction based on the dopamine level) in Example 3;
FIG. 6 illustrates a graph showing the results of a platform test (platform arrival time) in the animal experiment in Example 4;
FIG. 7 illustrates a graph showing the results of a probe test (the number of times to cross the former platform location) in the animal experiment in Example 4; and
FIG. 8 illustrates a graph showing the results of a probe test (time spent on the former platform location) in the animal experiment in Example 4.

### DETAILED DESCRIPTION

The present disclosure will be described in detail below.

### <Grape Pericarp Extract>

The grape pericarp extract of the present disclosure comprises 15 mg/g or more of malvidin-3,5-diglucoside in dry mass.

### (Grape Pericarp)

From the viewpoint of the malvidin-3,5-diglucoside content, the grape pericarp is preferably a grape pericarp of *Vitis Coignetiae, Vitis flexuosa,* a cross species between *Vitis Coignetiae* and *Vitis flexuosa,* a cross species between *Vitis Coignetiae* and *Vitis Vinifera,* or a cross species between *Vitis flexuosa* and *Vitis Vinifera,* with a grape pericarp of a cross species between *Vitis Coignetiae* and *Vitis Vinifera* or a cross species between *Vitis flexuosa* and *Vitis Vinifera* being more preferred.

### (Composition of Anthocyanins)

The grape pericarp extract comprises 15 mg/g or more of malvidin-3,5-diglucoside in dry mass. From the viewpoint of neuronal function modulation, the malvidin-3,5-diglucoside content is preferably 20 mg/g or more, and more preferably 25 mg/g or more. The upper limit of the malvidin-3,5-diglucoside content is not particularly limited and can be 500 mg/g or less, e.g., 100 mg/g.

The grape pericarp extract preferably comprises delphinidin-3-glucoside. From the viewpoint of complementarity of neuronal function modulating effects, the delphinidin-3-glucoside content is preferably 0.01 moles or more, and more preferably 0.1 moles or more, per mole of malvidin-3,5-diglucoside. Further, from the viewpoint of the balance of neuronal function modulating effects, the delphinidin-3-glucoside content is preferably 1.5 moles or less, and more preferably 1.0 mole or less, per mole of malvidin-3,5-diglucoside.

The grape pericarp extract preferably comprises malvidin-3-glucoside. From the viewpoint of complementarity of neuronal function modulating effects, the malvidin-3-glucoside content is preferably 0.01 moles or more, and more preferably 0.03 moles or more, per mole of malvidin-3,5-diglucoside. Further, from the viewpoint of the balance of neuronal function modulating effects, the malvidin-3-glucoside content is preferably 1.5 moles or less, and more preferably 1.0 mole or less, per mole of malvidin-3,5-diglucoside.

The grape pericarp extract can comprise malvidin-3-coumaroylglucoside. The malvidin-3-coumaroylglucoside content can be 0.01 moles or more and 2.0 moles or less, and is preferably 0.05 moles or more and 1.5 moles or less, per mole of malvidin-3,5-diglucoside.

The grape pericarp extract can comprise malvidin-3-coumaroylglucoside-5-glucoside. The malvidin-3-coumaroylglucoside-5-glucoside content can be 0.01 moles or more and 2.0 moles or less, and is preferably 0.05 moles or more and 1.5 moles or less, per mole of malvidin-3,5-diglucoside.

The grape pericarp extract can comprise resveratrol. The resveratrol content can be 0.001 moles or more per mole of malvidin-3,5-diglucoside, e.g., 0.003 moles or more and can be, for example, 0.100 moles or less.

### (Use)

The grape pericarp extract of the present disclosure exhibits superior neuronal function modulating effects. The term "neuronal function modulating effects" refer to effects related to the prevention of deterioration, retention, restoration, improvement of a neural function, or prevention of diseases related to a neural function. It is suggested in Examples described below that the grape pericarp extract of the present disclosure is effective for neuronal cell protection and also exhibits anti-inflammatory effects and inhibitory effects on neurotransmitter level reductions. While the detailed mechanism of the foregoing remains elusive, the actions of malvidin-3,5-diglucoside and other ingredients contained in the grape pericarp extract are considered to be involved.

The grape pericarp extract of the present disclosure is effective for neurodegenerative diseases and other various conditions associated with neuronal cell damage or neuronal cell reduction. The grape pericarp extract is particularly effective for antiaging. Specifically, the grape pericarp extract of the present disclosure can be used for the purpose of prevention or improvement of Alzheimer's disease, Parkinson's disease, depression or other neurodegenerative diseases, as well as for anti-stress and anti-fatigue. It should be noted that the grape pericarp extract of the present disclosure can also be used for healthy individuals, e.g., for the purpose of improving neural functions such as learning ability, memory ability, and reflection and reaction ability of healthy individuals. The grape pericarp extract of the present disclosure can be for anti-aging, anti-stress, anti-fatigue, restoration/improvement of learning ability, restoration/improvement of memory ability, and restoration/improvement of reflection and reaction ability.

Examples of subjects to be administered with the grape pericarp extract of the present disclosure include mammals, e.g., humans, domestic animals (e.g., horses, cows, pigs), pet animals (e.g., dogs, cats), and experimental (test) animals (e.g., mice, rats), with humans being preferred.

The dose of the grape pericarp extract of the present disclosure can be appropriately determined in view of species, age, body weight, conditions to be treated etc., of the subject. For example, when administering to a human, the dose can be 0.2 mg/day or more and 100 mg/day or less per kg of the body weight of the subject, preferably 0.5 mg/day or more, more preferably 1.0 mg/day or more, and preferably 40 mg/day or less, more preferably 20 mg/day or less, per kg of the body weight of the subject. When the subject is not a human, the dose can be determined appropriately by increasing or decreasing the values described above. Administration may be once or two or more times per day. The administration period can be appropriately determined in view of species, age, body weight, conditions to be treated etc., of the subject, but continuous use is preferred.

### (Production Method)

The grape pericarp extract can be obtained from a grape pericarp by extracting an ingredient containing malvidin-3,5-diglucoside using an organic solvent.

As the grape pericarp, a dried grape pericarp product can be used. Examples thereof include a dried product obtained by drying a grape pericarp by warm or hot air drying, and a dried product obtained by lyophilization of a grape pericarp.

Extraction is preferably performed using an acid-containing organic solvent or a mixed solvent of an acid-containing organic solvent and water. Preferred acids are strong acids and examples include hydrochloric acid, sulfuric acid, formic acid, and trifluoroacetic acid, with hydrochloric acid and formic acid, which are volatile acids, being preferred. Preferred organic solvents are polar solvents and examples thereof include methanol, ethanol, acetone, and tetrahydrofuran, with methanol and ethanol being preferred. The acid content can be adjusted as appropriate.

Extraction can be carried out by homogenizing a solution containing a grape pericarp or using a high-speed high-pressure extractor. From the viewpoint of extraction efficiency, the solution is preferably obtained by performing treatment under a high pressure (e.g., 5MPa to 15MPa s) condition. The resulting solution is optionally filtered and then the filtrate is concentrated and dried (e.g., by warm or hot air drying or lyophilization) to give a grape pericarp extract. Alternatively, the grape pericarp extract may be obtained as follows: the resulting solution is centrifuged, after which the supernatant is collected, a separation solvent is added to the supernatant to separate the aqueous layer containing a grape pericarp extract, and the aqueous layer is concentrated and dried (e.g., by warm or hot air drying or lyophilization). The separation solvent is not particularly limited as long as it is an organic solvent whose phase separates from water. Examples of such organic solvents include ethyl acetate, toluene, butyl acetate, hexane, heptane, dichloromethane, and cyclopentyl methyl ether.

### <Food Composition>

According to the present disclosure, there is provided a food composition that comprises the grape pericarp extract described above. The food composition may be a functional food, a dietary supplement, a health supplement, a nutritionally fortified food, a food with a functional claim, a food for special dietary use, a food for specified health use, or a food with a nutritional function.

The food composition can comprise ingredients acceptable in the production of foods. Examples thereof include carriers, excipients (e.g., lactose, sucrose, dextrin, hydroxypropylcellulose, polyvinylpyrrolidone), disintegrating agents, buffers, emulsifiers, suspending agents, stabilizers, preservatives, solvents (e.g., water, saline, and organic solvents such as ethanol). Foods can also comprise proteins, carbohydrates, lipids, vitamins, minerals, organic acids, organic bases, flavors, and other ingredients.

The food composition can be in any form including solids, liquids, mixtures, suspensions, pastes, gels, powders, and capsules. The food composition can be a beverage, confectionery, seasoning, daily dish or processed food. The food composition may also be a supplement. The food composition is preferably a beverage, and more preferably a beverage having an alcohol by volume of less than 3%. The food composition can be obtained by blending the grape pericarp extract into a food.

Diseases and conditions to be treated, administration regimen (subject, dose, period) etc. of the food composition can be the same as those for the grape pericarp extract described above. Specifically, the food composition can be for anti-aging, anti-stress, anti-fatigue, restoration/improvement of learning ability, restoration/improvement of memory ability, or restoration/improvement of reflection and reaction ability. The dose of the food composition can be such that the dose of the grape pericarp extract equals to the dose described above for the grape pericarp extract.

### <Pharmaceutical Composition>

The present disclosure also relates to a pharmaceutical composition that comprises the grape pericarp extract described above. The pharmaceutical composition can be a pharmaceutical or quasi-pharmaceutical agent.

The pharmaceutical composition can comprise a pharmaceutically acceptable ingredient. Examples thereof include carriers, excipients (e.g., lactose, sucrose, dextrin, hydroxypropylcellulose, polyvinylpyrrolidone), disintegrating agents, buffers, emulsifiers, suspending agents, stabilizers, preservatives, solvents (e.g., water, saline, and organic solvents such as ethanol). The pharmaceutical composition can also comprise other pharmacological components having a pharmacological activity.

The pharmaceutical composition can be used as a pharmaceutical or quasi-pharmaceutical agent. The pharmaceutical composition may be for oral or parenteral administration, but is preferably for oral administration. Dosage forms for oral administration can include powders, tablets, coating tablets, sugar-coated tablets, soft or hard capsules, solutions, emulsions, and suspensions. Dosage forms for parenteral administration can include suppositories, injections, infusions, ointments, creams, and gels.

Diseases and conditions to be treated, administration regimen (subject, dose, period) etc. of the pharmaceutical composition can be the same as those for the grape pericarp extract described above. Specifically, the pharmaceutical composition can be for anti-aging, anti-stress, anti-fatigue, restoration/improvement of learning ability, restoration/improvement of memory ability, or restoration/improvement of reflection and reaction ability. The dose of the pharmaceutical composition can be such that the dose of the grape pericarp extract equals to the dose described above for the grape pericarp extract.

### EXAMPLES

The present disclosure will be described in more detail below with reference to Examples, which however shall not be construed as limiting the scope of the present disclosure.

### <Sample Preparation>

Grapes with the following varietal names were used:
- Fujinoyume (cross species between Gyojano-mizu *(Vitis flexuosa)* and Merlot *(Vitis Vinifera))*
- Eight Gold (cross species between Gyojano-mizu *(Vitis flexuosa)* and Pinot noir *(Vitis Vinifera)*
- Pione (hybrid)
- Kaiji (hybrid)
- Muscat Bailey A (hybrid)

Extraction methods described below were used for sample preparation.

### - Extraction Method 1

Grapes were squeezed and the fruit juice was removed. Pericarps were placed on a stainless steel net cage so as not to come into contact with each other, and dried using a dryer by applying 70°C warm air to give a dried grape pericarp product. To 6 g of the dried grape pericarp product was added 40 mL of 2% formic acid in methanol and water (methanol : ultrapure water : formic acid = 70:28:2 (v/v/v)) and the mixture was stirred (360 rpm) for 12h at room temperature under light-shielded conditions to give an extract solution. The extract solution was filtered through filter paper (ADVANTEC quantitative filter paper No.5A, 110 mm), concentrated and lyophilized to give a grape pericarp extract powder.

### - Extraction Method 2

Grape pericarps (of 6-7 grapes) were placed in a stainless steel strainer and washed with tap water with a care not to crush the pericarps. The washed pericarps were placed in a thick zipped plastic bag for lyophilization. 1 g of the lyophilized pericarp was set in a high-speed high-pressure extractor (Nippon BUCHI, E-914) and extracted 3 times with 1% formic acid in methanol (methanol : formic acid = 99:1 (v/v)) under a high pressure of 10MPa to give an extract solution. About 100 mL of the obtained extract solution was concentrated and lyophilized using a rotary evaporator to give a grape pericarp extract powder.

The grape pericarp extract powder was dissolved in methanol and subjected to high-performance liquid chromatography-mass spectrometry (HPLC/MS) to quantify malvidin-3,5-diglucoside, malvidin-3-glucoside, delphinidin-3-gluco side, malvidin-3-coumaroylglucoside, malvidin-3-coumaroylglucoside-5-glucoside, and resveratrol contained in 1 g of the extract powder. The ingredients were identified and quantified using LC-MS libraries and by comparison with the measured values of standards. The results are as descried below.

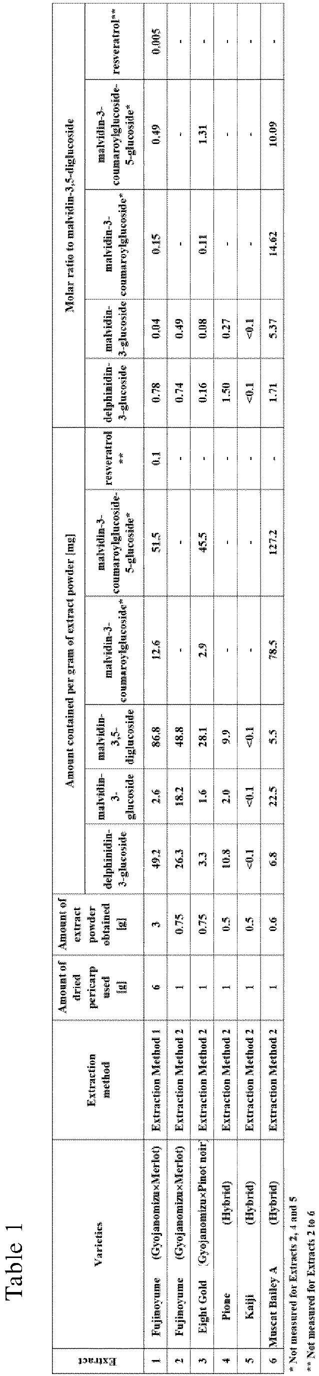

Extracts 1, 4, and 6 were used to prepare Samples 1 to 4 below.
Sample 1: Extract 1 was dissolved in 70% ethanol solution (ethanol : water = 70:30 (v/v)) and the concentration of the extract powder in the obtained solution was adjusted to 100 µg/mL. The concentration of malvidin-3,5-diglucoside was 12.56 µM (calculated as chloride).
Sample 2: Extract 4 was dissolved in 70% ethanol solution (ethanol : water = 70:30 (v/v)) and the concentration of the extract powder in the obtained solution was adjusted to 100 µg/mL. The concentration of malvidin-3,5-diglucoside was 1.43 µM (calculated as chloride).
Sample 3: Extract 6 was dissolved in 70% ethanol solution (ethanol : water = 70:30 (v/v)) and the concentration of the extract powder in the obtained solution was adjusted to 100 µg/mL. The concentration of malvidin-3,5-diglucoside was 0.80 µM (calculated as chloride).
Sample 4: Extract 1 was dissolved in ultrapure water (Milli-Q^{®} water) and the concentration of the extract powder in the obtained solution was adjusted to 7.5 mg/mL. The concentration of malvidin-3,5-diglucoside in the solution was 651 µg/mL (calculated as chloride).

### <Example 1: Cell Survival Experiment

Inhibitory effects of the grape pericarp extracts on neuronal cell death were confirmed by the experiment described below.

Each well of a 96-well plate (BD Biocoat) was seeded with SH-SY5Y cells (model of neuronal cells, ATCC) at a concentration of 2×10⁵ cells/mL (100 µL/well) and the cells were cultured at 37°C and 5%CO₂ for 24h. Culture medium used was F12/DMEM (Gibco) supplemented with 1% non-essential amino acids (Gibco), 15%FBS (Bio West), and 1% penicillin/streptomycin (Lonza).

After 24h, the culture medium was completely removed with an aspirator, 100 µL of culture medium Opti-MEM (Gibco) was added to each well, and the cells were cultured at 37°C and 5%CO₂ for 24h.

Opti-MEM was completely removed with an aspirator, and 110 µL of MTT reagent (3-(4,5-dimethylthiazol-2-yl)-2,5-diphenyltetrazolium bromide, 5 mg/mL) mixed with Opti-MEM (MTT reagent : Opti-MEM = 1:10) was added to each well and the cells were cultured at 37°C and 5%CO₂ for 24h. 100 µL of 10% SDS (sodium dodecyl sulfate) aqueous solution was added to each well and the cells were cultured at 37°C and 5%CO₂ for 24h.

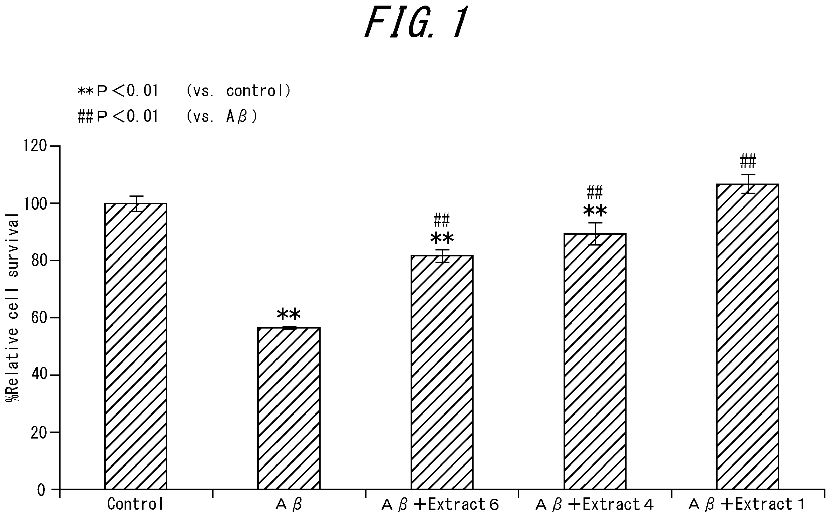

Absorbance at 570 nm was then measured on a microplate reader (Sumitomo Dainippon Pharma) to measure the MTT formazan production to calculate %relative cell survival (designated as "Control" in FIG. 1).

24h after seeding a 96-well plate (BD Biocoat) with SH-SY5Y cells, the culture medium was completely removed with an aspirator, 100 µL of Opti-MEM (Gibco) mixed with amyloid β protein (Beta-Amyloid 1-42, AnaSpec) so that the final concentration was 15 µM was added to each well, and the cells were cultured at 37°C and 5%CO₂ for 72h to induce cell death. Except for performing the procedure described above, the MTT formazan production was measured as described above to calculate %relative cell survival (designated as "Aβ" in FIG. 1).

24h after seeding a 96-well plate (BD Biocoat) with SH-SY5Y cells, the culture medium was completely removed with an aspirator, 90 µL of Opti-MEM mixed with Sample 1 so that the final concentration was 200 µg/mL was added to each well and allowed to stand for 10 min, 10 µL of Opti-MEM mixed with amyloid β protein (Beta-Amyloid 1-42, AnaSpec) so that the final concentration was 150 µM was added to each well, and the cells were cultured at 37°C and 5%CO₂ for 72h to induce cell death. Except for performing the procedure described above, the MTT formazan production was measured to calculate %relative cell survival (designated as "Aβ + Extract 1" in FIG. 1).

Except that Sample 2 was used instead of Sample 1, %relative cell survival (designated as "Aβ + Extract 4" in FIG. 1) was calculated in the same manner as described above.

Except that Sample 3 was used instead of Sample 1, %relative cell survival (designated as "Aβ + Extract 6" in FIG. 1) was calculated in the same manner as described above.

Comparison between "Control" and "Aβ" in FIG. 1 reveals that %relative cell viability was remarkably low in the latter compared to the former, indicating that cell death was induced by treatment with amyloid β protein.

"Aβ + Extract 1" in the drawing resulted in a significantly high %relative cell survival compared to "Aβ" and hence indicates that Extract 1 has inhibitory effects on cell death. %Relative cell survival of "Aβ + Extract 1" is comparable to that of "Control," suggesting that Extract 1 may have cell proliferation effects in addition to cell death inhibitory effects. On the other hand, %relative cell survival of "Aβ + Extract 4" and "Aβ + Extract 6" improved over "Aβ," but did not reach the level of "Aβ + Extract 1."

### <Example 2: Animal Experiment (Experiment for Confirming Anti-inflammatory Effects)>

Anti-inflammatory effects of the grape pericarp extracts were confirmed by the experiment described below.

Mice and administration regimens used in the experiment are as described below. Breeding conditions of mice are common to all groups.

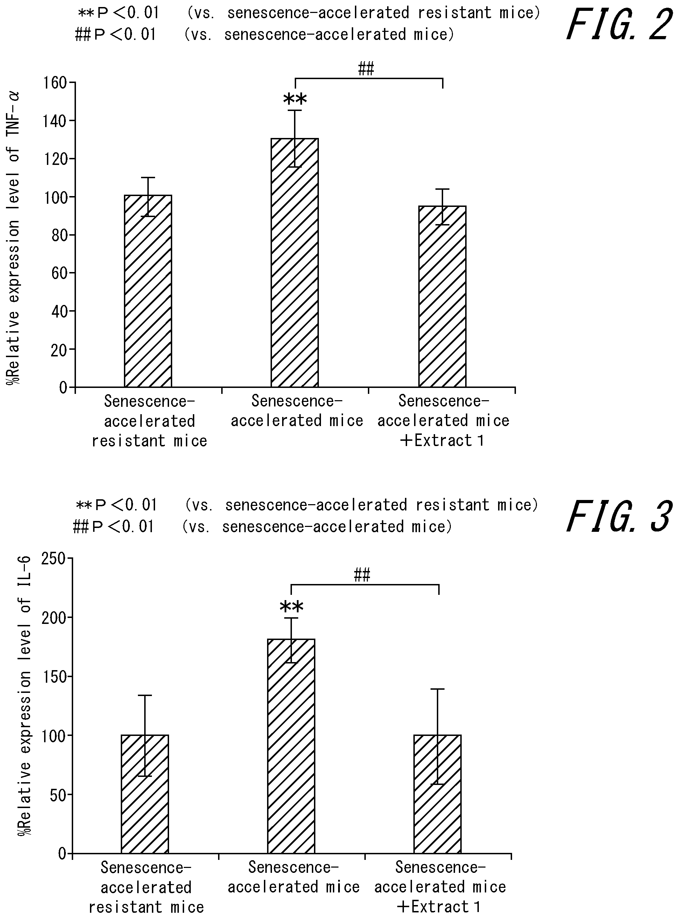

Extract-treated (SAMP8) group: Sample 4 was orally administered to 10 senescence-accelerated mice (SAMP8) (16 weeks old, weighing 28-30 g, Japan SLC, Inc.) at a daily dose of 3.34 mL per kg of mouse body weight for a period of 38 days (designated as "Senescence-accelerated mice + Extract 1" in FIGS. 2 and 3). 3.34 mL of Sample 4 contained 25 mg of extract powder, which corresponds to 2.17 mg of malvidin-3,5-diglucoside.

The human equivalent dose for 25 mg of extract powder per kg of mouse body weight is 2.0 mg of extract powder per kg of human body weight.

The human equivalent dose for 2.17 mg of malvidin-3,5-diglucoside per kg of mouse body weight is 0.18 mg of malvidin-3,5-diglucoside per kg of human body weight.

Water-treated (SAMP8) group: Ultrapure water (Milli-Q^{®} water) of almost the same volume as that of Sample 4 administered to the extract-treated (SAMP8) group was orally administered to 10 senescence-accelerated mice (SAMP8) (16 weeks old, weighing 28-30 g, Japan SLC, Inc.) for a period of 38 days (designated as "Senescence-accelerated mice" in FIGS. 2 and 3).

Water-treated (SAMR1) group: Ultrapure water (Milli-Q^{®} water) of almost the same volume as that of Sample 4 administered to the extract-treated (SAMP8) group was orally administered to 10 senescence-accelerated resistant mice (SAMR1) (16 weeks old, weighing 28.5-34 g, Japan SLC, Inc.) for a period of 38 days (designated as "Senescence-accelerated resistant mice" in FIGS. 2 and 3).

The content of the animal experiment is as described below.

The day after the completion of oral administration, the cerebral cortex was extracted from the mice of each group, and RNA was extracted and analyzed by real-time PCR for the expression of TNF-α (tumor necrosis factor α) and IL-6 (interleukin-6).

For RNA extraction, sample was obtained by adding ISOGEN Reagent (Nippon Gene Co., Ltd.) to the excised cerebral cortex followed by homogenization with a homogenizer. RNA extraction was performed according to the protocol recommended by Nippon Gene Co., Ltd. (see URL: see https://www.nippongene.com/siyaku/product/extraction/tds/tds#isogen-rna-dn a-protein.pdf).

Real-time PCR was performed according to the protocol recommended by Thermo Fisher Scientific using a reagent kit for TaqMan Real-Time PCR (Thermo Fisher Scientific) (see URL: https://assets.thermofisher.com/TFS-Assets/LSG/manuals/4304449#TaqManP CRMM#UG.pdf).

As shown in FIGS. 2 and 3, senescence-accelerated mice showed significant increases in the expression of the inflammatory cytokines TNF-α and IL-6 compared to senescence-accelerated resistant mice. On the other hand, in senescence-accelerated treated with the extract, the expression was suppressed to a level comparable to that in senescence-accelerated resistant mice.

### <Example 3: Animal Experiment (Experiment for Confirming Inhibitory Effects on Neurotransmitter Level Reduction)>

Inhibitory effects of the grape pericarp extracts on neurotransmitter level reduction were confirmed by the experiment described below.

Mice and administration regimens used in the experiment are as described below. Breeding conditions of mice are common to all groups.

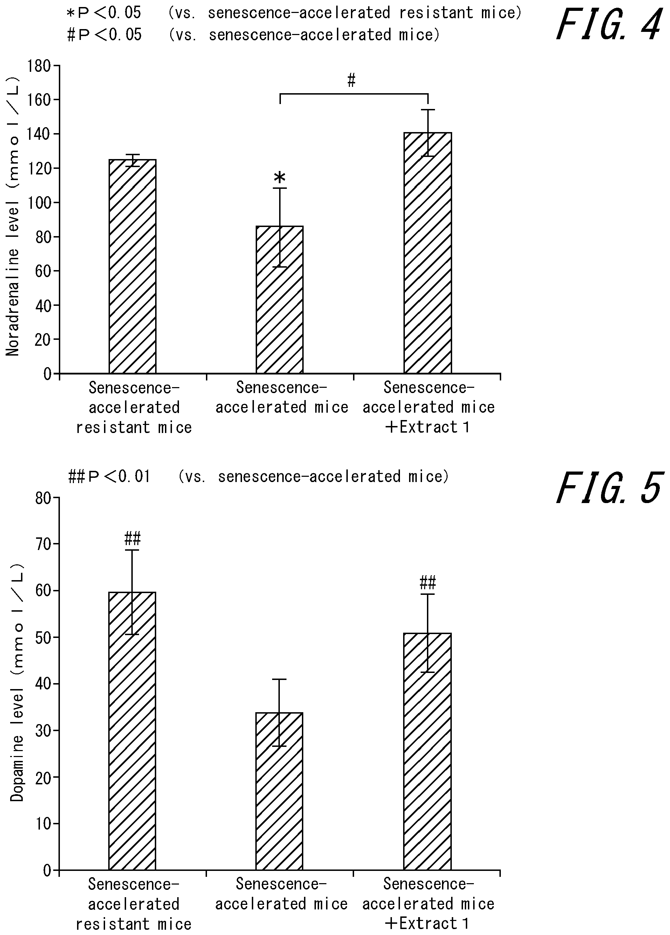

Extract-treated (SAMP8) group: Sample 4 was orally administered to 10 senescence-accelerated mice (SAMP8) (16 weeks old, weighing 28-30 g, Japan SLC, Inc.) at a daily dose of 3.34 mL per kg of mouse body weight for a period of 38 days (designated as "Senescence-accelerated mice + Extract 1" in FIGS. 4 and 5).

Water-treated (SAMP8) group: drinking water of almost the same volume as that of Sample 4 administered to the extract-treated (SAMP8) group was orally administered to 10 senescence-accelerated mice (SAMP8) (16 weeks old, weighing 28-30 g, Japan SLC, Inc.) for a period of 38 days (designated as "Senescence-accelerated mice" in FIGS. 4 and 5).

Water-treated (SAMR1) group: drinking water of almost the same volume as that of Sample 4 administered to the extract-treated (SAMP8) group was orally administered to 10 senescence-accelerated resistant mice (SAMR1) (16 weeks old, weighing 28.5-34 g, Japan SLC, Inc.) for a period of 38 days (designated as "Senescence-accelerated resistant mice" in FIGS. 4 and 5).

The content of the animal experiment is as described below.

The day after the completion of oral administration, the cerebral cortex was removed from the mice of each group. RIPA Lysis Buffer System (Santa Cruz Biotechnology, Inc.) was added to the cerebral cortex, and the cortex was homogenized with a homogenizer for protein extraction. Subsequently, centrifugation (10,000×g, 30min) was performed and the supernatant was collected and quantified for noradrenaline and dopamine.

Quantitation of noradrenaline was performed using an enzyme-linked immunosorbent assay (ELISA) kit (BAE-5200, LDN). Absorbance at 450 nm was measured and the noradrenaline level was calculated using a standard curve of known concentration of noradrenaline.

Quantitation of dopamine was performed using an enzyme-linked immunosorbent assay (ELISA) kit (BAE-5300, LDN). Absorbance at 450 nm was measured and the dopamine level was calculated using a standard curve of known concentration of dopamine.

As shown in FIGS. 4 and 5, senescence-accelerated mice showed significant reductions in the levels of the neurotransmitters noradrenaline and dopamine compared to senescence-accelerated resistant mice. On the other hand, the gene expression levels in senescence-accelerated mice treated with the extract were comparable to those in senescence-accelerated resistant mice.

### <Example 4: Animal Experiment (Experiment for Confirming effects on Improving Learning and Memory Ability)>

Effects of the grape pericarp extract administration on improving the learning and memory ability in animals were confirmed by the experiment described below.

Mice and administration regimens used in the experiment are as described below. Breeding conditions of mice are common to all groups.

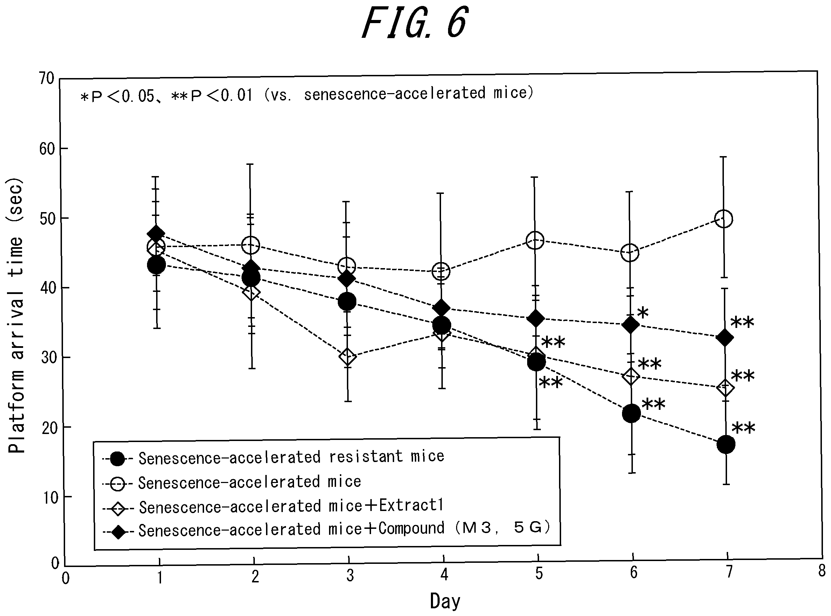

Extract-treated (SAMP8) group: Sample 4 was orally administered to 10 senescence-accelerated mice (SAMP8) (16 weeks old, weighing 28-30 g, Japan SLC, Inc.) at a daily dose of 3.34 mL per kg of mouse body weight for a period of 30 days (designated as "Senescence-accelerated mice + Extract 1" in FIGS. 6 to 8).

Water-treated (SAMP8) group: drinking water of almost the same volume as that of Sample 4 administered to the extract-treated (SAMP8) group was orally administered to 10 senescence-accelerated mice (SAMP8) (16 weeks old, weighing 28-30 g, Japan SLC, Inc.) for a period of 30 days (designated as "Senescence-accelerated mice" in FIGS. 6 to 8).

Water-treated (SAMR1) group: drinking water of almost the same volume as that of Sample 4 administered to the extract-treated (SAMP8) group was orally administered to 10 senescence-accelerated resistant mice (SAMR1) (16 weeks old, weighing 28.5-34 g, Japan SLC, Inc.) for a period of 30 days (designated as "Senescence-accelerated resistant mice" in FIGS. 6 to 8).

Compound (M3,5G)-treated (SAMP8) group: for reference, malvidin-3,5-diglucoside (FUJIFILM Wako Pure Chemical Industries, Ltd.) alone was orally administered to 10 senescence-accelerated mice (SAMP8) (16 weeks old, weighing 28-30 g, Japan SLC, Inc.) at a daily dose of 2.17 mg per kg of mouse body weight for a period of 30 days (designated as "Senescence-accelerated mice + Compound (M3,5G)" in FIGS. 6 to 8).

The content of the animal experiment is as described below.

From the day following the completion of oral administration, mice in each group were subjected to the Morris water maze test (platform test, probe test). A water pool having a diameter of 120 cm and a depth of 45 cm was prepared as the Morris water maze. A transparent platform was placed under the water surface of the pool. On the rim of the pool were four marks placed at equal intervals.

### Platform Test:

Mice were released into the pool and the time taken for the mice to arrive at the platform was measured. The time limit was set to 60 sec, with the arrival time when the mouse failed to arrive at the platform within 60 sec set to 60 sec. After arrival the mice were allowed to remain on the platform for 15 sec. The mouse from each group was subjected to this experiment once a day for 7 consecutive days. The results are shown in FIG. 6.

### Probe Test:

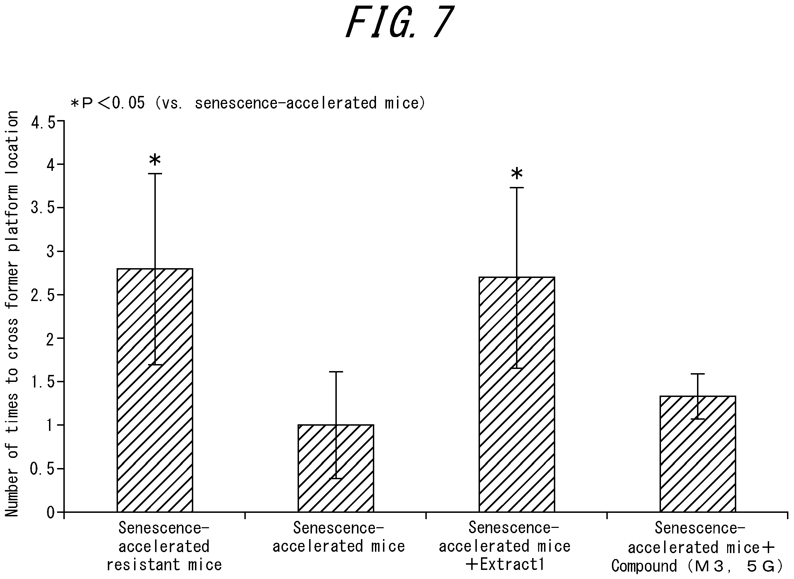

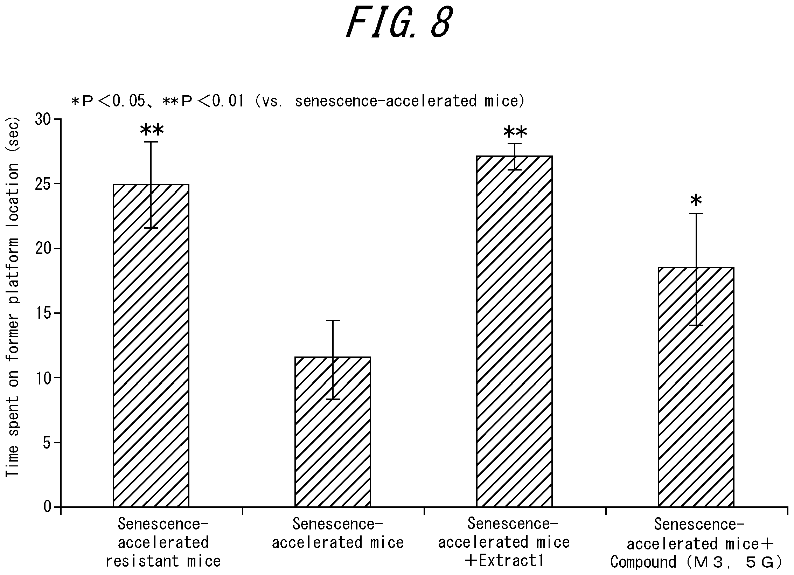

On the last day of the test, the platform was removed from the pool and the mice were again released into the pool. The number of times the mouse crossed the former platform location was counted and the time the mouse spent on the former platform location was measured. The results are shown in FIGS. 7 and 8.

The platform test assesses the spatial learning and memory of a mouse based on the time it took for the mouse to arrive at the platform by relying on visual stimuli such as landmarks. As shown in FIG. 6, the arrival time was shortened for senescence-accelerated resistant mice (water-treated), whereas the arrival time was not shortened for senescence-accelerated mice (water-treated). Senescence-accelerated mice treated with the extract exhibited arrival time shortening comparable to that for senescence-accelerated resistant mice (water-treated). When compared with senescence-accelerated mice treated with malvidin-3,5-diglucoside, senescence-accelerated mice treated with Extract 1 exhibited greater arrival time shortening.

The probe test assesses the memory retention ability of a mouse by the number of times the mouse crossed the former platform location and the time spent on the former platform location. As shown in FIGS. 7 and 8, senescence-accelerated mice (water-treated) crossed the former platform location significantly fewer times and spent shorter time on the former platform location compared to senescence-accelerated resistant mice (water-treated). For senescence-accelerated mice treated with Extract 1, the number of the times the mouse crossed the former platform location and the time spent on the former platform location were comparable to those for senescence-accelerated resistant mice (water-treated). When compared with senescence-accelerated mice treated with malvidin-3,5-diglucoside, senescence-accelerated mice treated with the extract crossed the former platform location more times and spent longer time on the former platform location.

As described above, improvements in the learning and memory ability of animals by administration of the extract of the present disclosure have been confirmed.

### INDUSTRIAL APPLICABILITY

The grape pericarp extract of the present disclosure is effective for neuronal cell protection and exerts neuronal function modulating effects through neuronal cell protection and is expected to improve and prevent neurodegenerative diseases such as Alzheimer's disease, Parkinson's disease, and depression. The grape pericarp extract is therefore expected to be used for health supplements and other foods, pharmaceuticals etc. to expand the related markets. Because the grape pericarp extract of the present disclosure is of natural origin, it is considered safe and suitable for daily or continuous ingestion. Thus, the present disclosure has high industrial utility.

## Claims

1. A grape pericarp extract comprising 15 mg/g or more of malvidin-3,5-diglucoside in dry mass.

2. The grape pericarp extract according to claim 1, comprising delphinidin-3-glucoside.

3. The grape pericarp extract according to claim 2, wherein delphinidin-3-glucoside is contained in an amount of 0.01 moles or more and 1.5 moles or less per mole of malvidin-3,5-diglucoside.

4. The grape pericarp extract according to any one of claims 1 to 3, comprising malvidin-3-glucoside.

5. The grape pericarp extract according to claim 4, wherein malvidin-3-glucoside is contained in an amount of 0.01 moles or more and 1.5 moles or less per mole of malvidin-3,5-diglucoside.

6. The grape pericarp extract according to any one of claims 1 to 5, comprising malvidin-3-coumaroylglucoside.

7. The grape pericarp extract according to claim 6, wherein malvidin-3-coumaroylglucoside is contained in an amount of 0.01 moles or more and 2.0 moles or less per mole of malvidin-3,5-diglucoside.

8. The grape pericarp extract according to any one of claims 1 to 7 containing malvidin-3-coumaroylglucoside-5-glucoside.

9. The grape pericarp extract according to claim 8, wherein malvidin-3-coumaroylglucoside-5-glucoside is contained in an amount of 0.01 moles or more and 2.0 moles or less per mole of malvidin-3,5-diglucoside.

10. The grape pericarp extract according to any one of claims 1 to 9, comprising resveratrol.

11. The grape pericarp extract according to claim 10, wherein resveratrol is contained in an amount of 0.001 moles or more per mole of malvidin-3,5-diglucoside.

12. The grape pericarp extract of any one of claims 1 to 11, wherein the grape pericarp is a pericarp of one or more types of grapes selected from the group consisting of *Vitis Coignetiae, Vitis flexuosa,* a cross species between *Vitis Coignetiae* and *Vitis flexuosa,* a cross species between *Vitis Coignetiae* and *Vitis Vinifera,* and a cross species between *Vitis flexuosa* and *Vitis Vinifera.*

13. A food composition comprising the grape pericarp extract according to any one of claims 1 to 12.

14. The food composition according to claim 13, wherein the food composition is a beverage having an alcohol by volume of less than 3%.

15. A pharmaceutical composition comprising the grape pericarp extract according to any one of claims 1 to 12.

16. A method for producing the grape pericarp extract according to any one of claims 1 to 12, wherein an ingredient comprising malvidin-3,5-diglucoside is extracted from a grape pericarp using an organic solvent.

17. The method according to claim 16, wherein a dried grape pericarp product is used as the grape pericarp and extraction is performed using either an acid-containing organic solvent or a mixed solvent of an acid-containing organic solvent and water.
